Europäisches Patentamt

European Patent Office

Office européen des brevets

(19)

(11) EP 0 870 064 B1

(12) FASCICULE DE BREVET EUROPEEN

(45) Date de publication et mention
de la délivrance du brevet:
**18.09.2002 Bulletin 2002/38**

(51) Int Cl.⁷: **C13F 1/02**, C13F 3/00,
C07H 3/04, C07H 1/06,
C13K 1/10, C13K 5/00

(21) Numéro de dépôt: **96941694.0**

(22) Date de dépôt: **04.12.1996**

(86) Numéro de dépôt international:
**PCT/FR96/01931**

(87) Numéro de publication internationale:
**WO 97/021838 (19.06.1997 Gazette 1997/26)**

(54) **SUCRES OU ALCOOLS DE SUCRES MICROCRISTALLINS; PROCEDE POUR LES PREPARER**

MIKROKRISTALLINE ZUCKER ODER ZUCKERALKOHLE;VERFAHREN ZUR HERSTELLUNG
DERSELBEN

MICROCRYSTALLINE SUGARS OR SUGAR-ALCOHOLS; METHOD FOR PREPARING THE SAME

(84) Etats contractants désignés:
**AT BE CH DE DK ES FI FR GB GR IE IT LI LU MC
NL PT SE**
Etats d'extension désignés:
**AL LT LV RO SI**

(30) Priorité: **11.12.1995 FR 9514643**

(43) Date de publication de la demande:
**14.10.1998 Bulletin 1998/42**

(73) Titulaire: **BEGHIN-SAY S.A.
59239 Thumeries (FR)**

(72) Inventeurs:
• **MAITRE, Jean-Paul
F-69970 Marennes (FR)**
• **MENTECH, Julio
F-69006 Lyon (FR)**
• **REYNAUD, Sylvie
F-69100 Villeurbanne (FR)**
• **WONG, Emile
F-01700 Neyron (FR)**

(74) Mandataire: **Grosset-Fournier, Chantal Catherine
Grosset-Fournier & Demachy s.a.r.l.,
20, rue de Maubeuge
75009 Paris (FR)**

(56) Documents cités:
EP-A- 0 039 123      EP-A- 0 052 413
EP-A- 0 052 919      WO-A-91/11179
DE-A- 1 910 752      DE-A- 3 842 751
FR-A- 2 244 411      FR-A- 2 669 511
US-A- 3 194 682      US-A- 3 365 331

**Description**

L'invention traile de compositions de sucre sous une forme cristalline, fluide et non montante. La présente invention concerne le domaine de la cristallisation du sucre, et plus précisément, elle décrit une méthode d'obtention de compositions de sucre cristallisé de granulométrie fine. L'invention décrit une composition de sucre cristallin de forme régulière, de granulométrie fine et bien définie.

Pendant la cristallisation, la répartition granulométrique des cristaux dépend principalement des processus suivants :

- la nucléation,
- la croissance des cristaux,
- l'attrition,
- l'agglomération,
- la maturation des cristaux.

Pour obtenir une grande quantité de cristaux réguliers et de granulométrie fine, il est nécessaire d'appliquer un procédé favorisant la nucléation plus que 1a croissance cristalline. Pour cela il est nécessaire d'utiliser les moyens appropriés permettant un bon contrôle des paramètres de cristallisation.

Les procédés de cristallisation existants ne permettent pas l'obtention directe d'une grande quantité de cristaux de sucre de forme régulière avec une granulométrie très fine. Dans la fabrication de divers types de sucre, un procédé a été développé, plus connu comme étant un procédé de transformation. Ce procédé est utilisé pour la production de sucre en poudre granulé, fluide, non mottant et facilement dispersable en solution aqueuse. Ce procédé a été abondamment décrit dans plusieurs brevets.

US 3,194,682 (Tippens et al.) décrit un procédé utilisant un sirop concentré à 95-97 brix (% en poids de matières sèches) à 121-129°C qui est soumis à un refroidissement rapide sous agitation énergique. Cette méthode permet 1a fabrication d'agglomérats dont les cristaux de sucre sont de taille fondant (3-50 microns).

US 3,365,331 (Miller et al.) décrit un procédé similaire qui conduit à la fabrication d'agglomérats. Dans ce cas, les cristaux sont obtenus par battage d'un sirop sursaturé.

Dans le brevet EP 0 052 413, le procédé de battage à une température bien contrôlée permet une incorporation de composés thermosensibles dans le produit final.

Tous les procédés décrits conduisent à une poudre de sucre fin granulé. Les granules sont de forme irrégulière donnant des poudres de basse densité. La sélection de la granulométrie se faisant par tamisage, le rendement en une classe de poudre est de ce fait faible. Il existe donc un besoin de développement d'un procédé permettant la fabrication avec de bons rendements de cristaux réguliers et de granulométrie fine, ce que permet la présente invention.

Plus précisément, l'invention a notamment pour objet une composition contenant des microcristaux de sucre caractérisée en ce que les cristaux de sucre obtenus sont de forme régulière, ne s'agglomèrent pas, et leur répartition granulométrique est de type gaussien autour d'une ouverture moyenne comprise entre 20 et 220 $\mu$m, notamment 20 et 200 $\mu$m, avec un coefficient de variation (CV) compris entre 20% et 50% ou leur répartition granulométrique est caractérisée par un indice d'uniformité compris entre 1 et 5, notamment entre 2.5 et 3.5.

La granulométrie est déterminée par tamisage sur une série de tamis normalisés (NF11-501) de 200 mm de diamètre.

Le coefficient de variation (CV) est calculé par la formule :

$$CV = 100 \times \sigma/O.M.,$$

dans laquelle $\sigma$ est l'écart type et O.M. est l'ouverture moyenne.

S'agissant de l'indice d'uniformité, il est obtenu par tamisage de la composition cristalline et calculé suivant la formule :

$$\frac{\text{Taille de particule correspondant à 60\% du passage de la poudre}}{\text{Taille de particule correspondant à 10\% du passage de la poudre}}$$

L'invention a aussi pour objet une méthode d'obtention d'une composition de sucre microcristallin caractérisée en ce que les cristaux ont une granulométrie moyenne comprise entre 20 et 220 $\mu$m, notamment 20 et 200 $\mu$m obtenus après les étapes suivantes :

a) fabrication d'un sirop concentré,

b) diminution de la pression,

c) évaporation sous pression réduite avec agitatio vigoureuse dans la zone de cristallisation jusqu'à l'apparition des cristaux,

d) arrêt de l'évaporation et maintien de l'agitation pendant un certain temps,

e) reprise de l'évaporation et de l'agitation jusqu'à l'obtention d'un produit sec,

la température du sirop étant maintenue de 40°C à 100°C, et notamment de 70°C à 100°C pendant la durée des étapes a) à e) décrites ci-dessus.

Ce procédé sera dans la suite désigné par "procédé I".

Selon un mode de réalisation avantageux, l'invention concerne une composition contenant des microcristaux de sucre caractérisée en ce que les cristaux sont essentiellement des monocristaux de forme géométrique régulière, ne présentant pas de brisure, homogènes les uns par rapport aux autres, et en ce que

- la granulométrie suit une distribution gaussienne dont la médiane est d'environ 20 à environ 220 $\mu$m, et notamment d'environ 20 $\mu$m à environ 200 $\mu$m, le coefficient de variation étant d'environ 20% à environ 50%, notamment d'environ 30% à 45%, ou d'environ 35% à 45%, ou d'environ 30% à 40% ou
- la répartition granulométrique est caractérisée par un indice d'uniformité compris entre 1 et 5, notamment entre 2.5 et 3.5.

Par "sucre", on désigne les mono-, di- et oligosaccharides, ainsi que les polyols obtenus par réduction de ceux-ci.

L'expression "monocristaux ne présentant pas de brisure" signifie que ces cristaux ne présentent pas d'angles aigus liés à une opération de broyage.

L'expression "homogènes les uns par rapport aux autres" signifie que ces cristaux sont de géométrie cristalline comparable.

Avantageusement, les monocristaux des compositions de l'invention ont une ouverture moyenne d'environ 80 $\mu$m à environ 120 $\mu$m.

La composition de l'invention est caractérisée en ce qu'elle présente les propriétés suivantes :

- sa vitesse de dissolution est d'environ 5 à environ 10, notamment d'environ 7 à environ 9 secondes, dans les conditions suivantes : 10 g de composition pour 100 ml d'eau pure déminéralisée, à la température de 18°C,
- elle est non mottante,
- son indice de coulabilité est supérieur à environ 80, et varie d'environ 80 à environ 85, notamment d'environ 81 à environ 82, mesuré selon le test d'Hosakawa, tel que décrit dans IRON WORKS, LTD, Osaka, Japon, et lorsqu'il s'agit du glucose, l'indice de coulabilité est d'environ 55 à environ 70,
- la densité du produit tassé est d'environ 0,90 à environ 1,00, notamment d'environ 0,97 à environ 1,00, et la densité du produit non tassé est d'environ 0,75 à environ 0,90, notamment d'environ 0,83 à environ 0,87, mesurées selon le test d'Hosakawa, et lorsque le susdit produit est du glucose, la densité du produit tassé est d'environ 0,70 à environ 0,90 et la densité du produit non tassé est d'environ 0,50 à environ 0,70.

L'expression "non mottante" signifie que les cristaux ne s'agglomèrent pas entre eux dans les conditions normales de température (10 à 30°C) et humidité (40 à 80 %) ambiantes.

Selon un autre mode de réalisation avantageux de l'invention, la composition est caractérisée en ce qu'elle contient des ingrédients additionnels, à raison d'environ 0% à environ 10%, et avantageusement à raison d'environ 5%, ces ingrédients étant de façon avantageuse choisis parmi les composés thermosensibles, des composés ayant des propriétés alimentaires ou pharmacologiques, ou des composés ayant un goût ou une couleur recherchés.

La composition de l'invention est susceptible d'être obtenue par le procédé comprenant les étapes suivantes :

a) on prépare un sirop de saccharose concentré, d'environ 60 à environ 97, notamment 75% en poids de matières sèches,

b) on réduit la pression qui passe de la pression atmosphérique à une valeur d'environ 100 à environ 300 mbars, notamment d'environ 200 mbars, pour commencer à évaporer une partie de l'eau contenue dans le sirop de sucre, le taux d'évaporation étant d'environ 20%,

c) on évapore une partie de l'eau contenue dans le sirop de sucre sous pression réduite (environ 200 mbars) et on brasse le sirop, notamment par agitation mécanique à une vitesse périphérique d'environ 100 à environ 350 m/mn, notamment 200 à 350 m/mn, jusqu'à atteindre un coefficient de sursaturation de sucre compris entre 1 et 1,3, notamment 1.1 et 1,3. et on provoque la cristallisation par une agitation vigoureuse du sirop (en plus du brassage susmentionné), notamment par chocs mécaniques générés par battage par impact, dans cette zone de sursaturation,

d) on effectue la suite de la cristallisation par arrêt de l'évaporation et de l'agitation vigoureuse (battage), et maintien d'une agitation régulière (brassage) pendant le temps nécessaire à l'obtention des cristaux de taille souhaitée, et avantageusement pendant environ 5 mn à environ 20 mn,

e) on reprend l'évaporation (toujours avec brassage du milieu à une vitesse d'environ 100 à environ 250 m/mn) jusqu'à l'obtention de cristaux contenant moins de 1%, notamment moins de 0,5% d'humidité,

la température étant maintenue à une valeur d'environ 70°C à environ 100°C, pendant toute la durée du procédé, et la pression étant avantageusement maintenue à environ 200 mbars pendant les étapes c) à e).

Selon un mode de réalisation avantageux de l'invention, le procédé I est caractérisé par les étapes suivantes :

a) on prépare un sirop de saccharose concentré, d'environ 60 à environ 97, notamment 75% en poids de matières sèches,

b) on réduit la pression qui passe de la pression atmosphérique à une valeur d'environ 100 à environ 300 mbars, notamment d'environ 200 mbars, pour commencer à évaporer une partie de l'eau contenue dans le sirop de sucre, le taux d'évaporation étant d'environ 20%,

c) on évapore une partie de l'eau contenue dans le sirop de sucre sous pression réduite (environ 200 mbars) et on brasse le sirop, notamment par agitation mécanique à une vitesse périphérique d'environ 100 à environ 350 m/mn. notamment 200 à 350 m/mn, jusqu'à atteindre un coefficient de sursaturation de sucre compris entre 1 et 1,3, notamment 1,1 et 1,3, et on provoque la cristallisation par une agitation vigoureuse du sirop (en plus du brassage susmentionné, notamment par chocs mécaniques générés par battage par impact, dans cette zone de sursaturation,

d) on effectue la suite de la cristallisation par arrêt de l'évaporation et de l'agitation vigoureuse (battage), et maintien d'une agitation régulière (brassage) pendant le temps nécessaire à l'obtention des cristaux de taille souhaitée, et avantageusement pendant environ 5 mn à environ 20 mn,

e) on reprend l'évaporation (toujours avec brassage du milieu à une vitesse d'environ 100 à environ 350 m/mn, notamment 200 à 350 m/mn jusqu'à l'obtention de cristaux contenant moins de 1%, notamment moins de 0,5% d'humidité,

la température étant maintenue à une valeur d'environ 40°C à environ 100°C, notamment d'environ 70°C à environ 100°C, pendant toute la durée du procédé, et la pression étant avantageusement maintenue à environ 200 mbars pendant les étapes c) à e).

Le procédé de l'invention commence avec la préparation du sirop concentré de sucre. La concentration adaptée est comprise, à titre indicatif, entre 60 et 80% en poids de matières sèches. Afin d'éviter la recristallisation et la dégradation du sucre ou tout autre produit ajouté à la solution, la température est maintenue de 40°C à 100°C, notamment de 70°C à 100°C. La pression est réduite à 100-300 mbars pour démarrer l'évaporation. En même temps, le sirop est maintenu sous agitation. Cette agitation mécanique ou brassage du sirop est nécessaire à l'homogénéisation du milieu, et est réalisée à l'aide d'un mobile d'agitation avantageusement placé en fond de cuve utilisée dans le procédé de l'invention, A titre d'illustration, ce brassage peut être réalisé avec un mélangeur-évaporateur, un cristalliseur, un mélangeur-homogénéiseur. un mélangeur-malaxeur ou tout autre équipement adapté. Il est important que ce brassage soit énergique et que l'énergie apportée au sirop soit contrôlée. En outre, pour le bon fonctionnement du procédé, l'installation doit pouvoir fonctionner sous pression réduite et température régulée.

L'agitation vigoureuse, avantageusement effectuée par battage par impact, de la solution stimule la formation de germes et un voile est observable après un certain temps. Ces conditions sont maintenues pendant quelques minutes et ensuite, l'évaporation est arrêtée. A titre d'illustration, les essais qui sont décrits dans les exemples de l'invention sont réalisés sur un évaporateur-mélangeur Guédu de 45 litres, équipé pour le battage par impact d'un mixeur ou de couteaux dont la vitesse de rotation est d'environ 1000 à environ 2000 tours/mn.

Le brassage est maintenu afin de mieux contrôler la croissance des cristaux. Pendant la phase finale. l'évaporation est poursuivie avec brassage jusqu'à l'obtention de cristaux secs.

La variation de la vitesse d'agitation pour le brassage du milieu, du taux d'évaporation et de la durée des différentes étapes, permet de préparer des cristaux d'une granulométrie moyenne bien définie pouvant être obtenus de façon reproductible.

La composition de l'invention est également susceptible d'être obtenue par un procédé comprenant les étapes suivantes :

a) on prépare un sirop concentré,

b) on évapore le sirop sous pression avec agitation vigoureuse dans la zone de cristallisation jusqu'à l'apparition des cristaux, avec contrôle de la température et du débit d'évaporation jusqu'à une teneur en matières sèches d'environ 80% à environ 90%,
c) on poursuit l'évaporation avec réduction de la vitesse d'agitation jusqu'à l'obtention d'un produit sec, la température étant maintenue constante par rapport à l'étape précédente,

la température étant ajustée et maintenue à une valeur déterminée dans l'intervalle d'environ 40°C à environ 100°C, et notamment d'environ 70°C à environ 100°C, pendant la durée des étapes a) à c) décrites ci-dessus.

La composition de l'invention est également susceptible d'être obtenue de la façon suivante :

a) on prépare un sirop de sucre concentré d'environ 60% à environ 97%, notamment 75% en poids de matières sèches,
b) on provoque l'évaporation du sirop par réduction de la pression de manière à atteindre l'ébullition de ce sirop à la température choisie,
c) on brasse le sirop notamment par agitation mécanique à une vitesse périphérique d'environ 100 à environ 350 m/mn, notamment 200 à 350 m/mn, le coefficient de sursaturation du sirop étant compris entre 1 et 1,3, notamment 1,1 et 1,3, et on provoque la cristallisation par une agitation vigoureuse du sirop (en plus du brassage susmentionné), notamment par chocs mécaniques générés par battage par impact, dans cette zone de sursaturation,
d) on poursuit l'évaporation dans les mêmes conditions de température et de pression que celles utilisées dans les étapes précédentes, jusqu'à obtention d'un milieu dont les cristaux constituent la phase majoritaire (supérieur à environ 50%, et notamment supérieur à environ 70% par rapport au milieu),

la vitesse d'agitation étant réduite d'environ 50 à environ 200 m/mn, la température étant maintenue constante par rapport aux étapes précédentes, le battage étant maintenu jusqu'à obtention d'un produit sec composé de cristaux de taille souhaitée contenant moins de 1%, notamment moins de 0,5% d'humidité,
la température étant ajustée et maintenue à une valeur constante dans la gamme allant d'environ 40°C à environ 100°C, notamment d'environ 70°C à environ 100°C, pendant toute la durée des étapes.

L'invention concerne également un procédé de préparation des compositions décrites ci-dessus, lequel procédé est caractérisé par les étapes suivantes :

a) on prépare un sirop concentré,
b) on évapore le sirop sous pression avec agitation vigoureuse dans la zone de cristallisation jusqu'à l'apparition des cristaux, avec contrôle de la température et du débit d'évaporation jusqu'à une teneur en matières sèches d'environ 80% à environ 90%,
c) on poursuit l'évaporation avec réduction de la vitesse d'agitation jusqu'à l'obtention d'un produit sec, la température étant maintenue constante par rapport à l'étape précédente,
la température étant ajustée et maintenue à une valeur déterminée dans l'intervalle d'environ 40°C à environ 100°C, et notamment d'environ 70°C à environ 100°C, pendant la durée des étapes a) à c) décrites ci-dessus.

Ce procédé sera dans la suite désigné par "procédé II".

Selon un mode de réalisation avantageux de l'invention, le procédé II est caractérisé par les étapes suivantes :

a) on prépare un sirop de sucre concentré d'environ 60% à environ 97%, notamment 75% en poids de matières sèches,
b) on provoque l'évaporation du sirop par réduction de la pression de manière à atteindre l'ébullition de ce sirop à la température choisie,
c) on brasse le sirop notamment par agitation mécanique à une vitesse périphérique d'environ 100 à environ 350 m/mn, notamment 200 à 350 m/mn, le coefficient de sursaturation du sirop étant compris entre 1 et 1.3, notamment 1,1 et 1,3, et on provoque la cristallisation par une agitation vigoureuse du sirop (en plus du brassage susmentionné), notamment par chocs mécaniques générés par battage par impact, dans cette zone de sursaturation,
d) on poursuit l'évaporation dans les mêmes conditions de température et de pression que celles utilisées dans les étapes précédentes, jusqu'à obtention d'un milieu dont les cristaux constituent la phase majoritaire (supérieur à environ 50%. et notamment supérieur à environ 70% par rapport au milieu),

la vitesse d'agitation étant réduite d'environ 50 à environ 200 m/mn. la température étant maintenue constante par rapport aux étapes précédentes, le battage étant maintenu jusqu'à obtention d'un produit sec composé de cristaux de taille souhaitée contenant moins de 1%, notamment moins de 0,5% d'humidité,
la température étant ajustée et maintenue à une valeur constante dans la gamme allant d'environ 40°C à environ 100°C, notamment d'environ 70°C à environ 100°C, pendant toute la durée des étapes.

Le procédé II commence avec la préparation du sirop concentré de sucre. La concentration adaptée est comprise, à titre indicatif, entre 60 et 80% en poids de matière sèche. Afin d'éviter la recristallisation et la dégradation du sucre ou tout autre produit ajouté à la solution, la température est maintenue de 40°C à 100°C, notamment de 70°C à 100°C. Le sirop est maintenu sous agitation et la pression est abaissée de manière à atteindre l'ébullition du sirop à la température choisie. Cette agitation mécanique ou brassage du sirop est nécessaire à l'homogénéisation du milieu et est réalisée à l'aide d'un mobile d'agitation avantageusement placé en fond de cuve utilisée dans le procédé de l'invention. A titre d'illustration, ce brassage peut être réalisé avec un mélangeur-évaporateur, un cristalliseur, un mélangeur-homogénéiseur, un mélangeur-malaxeur ou tout autre équipement adapté. Il est important que ce brassage soit énergique et que l'énergie apportée au sirop soit contrôlée. En outre, pour le bon fonctionnement du procédé, l'installation doit pouvoir fonctionner sous pression réduite et température régulée,

L'agitation vigoureuse avantageusement effectuée par battage et impact de la solution stimule la formation de germes et un voile est observable après un certain temps.

La concentration du sirop est conduite avec un débit d'évaporation compris entre 20 et 30% par heure de la quantité d'eau initiale. L'évaporation est effectuée sous pression réduite, pression définie par la température du sirop pour obtenir l'ébullition du milieu à cette température.

Le système est maintenu dans cet état d'équilibre débit d'évaporation/pression/température jusqu'à un taux d'évaporation de 65% environ.

Le milieu devient alors très pâteux et, dans cette deuxième étape, la vitesse d'agitation du mobile est abaissée à 190 m/mn (vitesse périphérique) et l'évaporation est poursuivie avec une pression décroissant progressivement pour maintenir une température constante jusqu'à obtention d'une poudre sèche.

Par rapport au procédé I, le procédé II présente les différences suivantes :

. suppression de l'étape d) : "arrêt de l'évaporation et maintien de l'agitation pendant un certain temps",
. le procédé II est avantageusement appliqué aux essais industriels.

Les exemples présentés illustrent l'application du procédé d'invention permettant la fabrication de compositions de cristaux de sucre ayant une taille moyenne comprise entre 80 et 150 µm (Exemples 1 et 2). Par ailleurs, un exemple décrit l'utilisation du procédé pour l'obtention de cristaux de sucre contenant un deuxième composé, en l'occurrence du caramel (Exemple 3).

Les exemples 4 à 6 décrivent respectivement la préparation de glucose, de lactose et d'érythritol selon l'invention.

L'exemple 7 correspond à un essai industriel.

La présente invention décrit une composition de sucre microcristallin dont l'ouverture moyenne est centrée autour de 20 à 220 µm, notamment 20 à 200 µm. La distribution de la taille des cristaux autour de la valeur moyenne est de type gaussien, avec un CV compris entre 20% et 50% ou son indice d'uniformité est compris entre 1 et 5. Les cristaux, de forme régulière, ne sont pas des agglomérats. Les cristaux ont une densité élevée. Le produit est fluide et se dissous rapidement dans l'eau. Les cristaux obtenus par cette méthode ne demandent pas de tamisage particulier autre que l'élimination des agglomérats et particules supérieurs à 300 µm représentant moins de 10% de la composition. La poudre est obtenue avec un bon rendement et une distribution de type gaussien ou présentant un indice d'uniformité compris entre 1 et 5. Comme le procédé pour la fabrication dudit produit est très bien contrôlé, il est possible d'obtenir des cristaux de granulométrie moyenne désirée en modifiant seulement certains paramètres. Par conséquent, la présente invention décrivant des compositions de sucre microcristallin de diamètre spécifique entre 20 et 220 µm et plus précisément entre 80 et 150 µm est bien démontrée.

Le procédé de la présente invention permet l'addition d'ingrédients désirés au sucre, l'ajout pouvant être fait dans le cadre du procédé I, préférentiellement après formation du voile et avant l'arrêt de l'évaporation, par exemple entre l'étape c) et l'étape d).

Dans le cadre du procédé II, l'ajout peut être fait au moment où la sursaturation atteint une valeur comprise entre 1,0 et 1,3.

On observe, dans ce cas, une co-cristallisation du sucre avec un autre ingrédient. La présente invention décrit également le sucre microcristallin de granulométrie moyenne souhaitée dopé avec un ou des ingrédients choisis. Une large gamme d'ingrédients tels que les gommes, émulsifiants, produits chimiques peuvent être ajoutés. Les cristaux de sucre servent dans ce cas de support pour des ingrédients valorisés, par exemple comme produits alimentaires ou pharmaceutiques, soit pour la couleur, soit pour le goût, ou pour toute autre propriété recherchée.

La présente invention décrit par conséquent des compositions de microcristaux de sucre et d'autres ingrédients.

Le procédé décrit dans la présente invention permet l'utilisation de conditions contrôlées de température. Ainsi, il est possible d'ajouter un second ingrédient thermosensible. Les composés thermosensibles peuvent être des vitamines, aminoacides, caroténoïdes, antibiotiques.

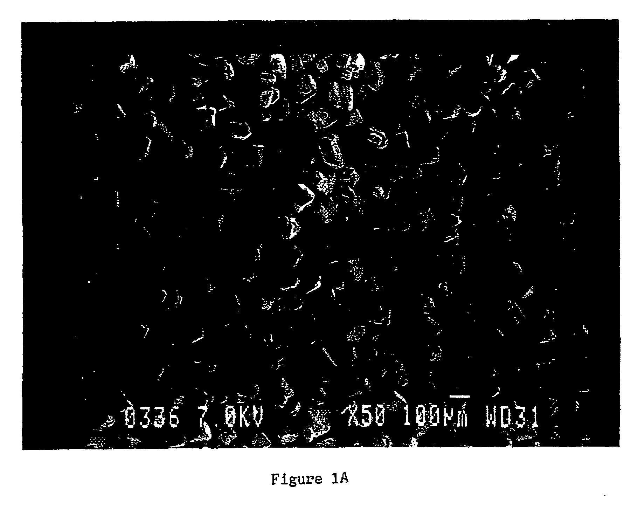

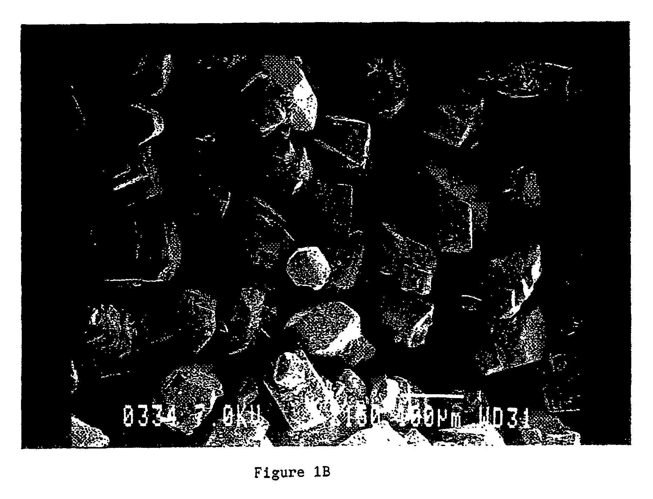

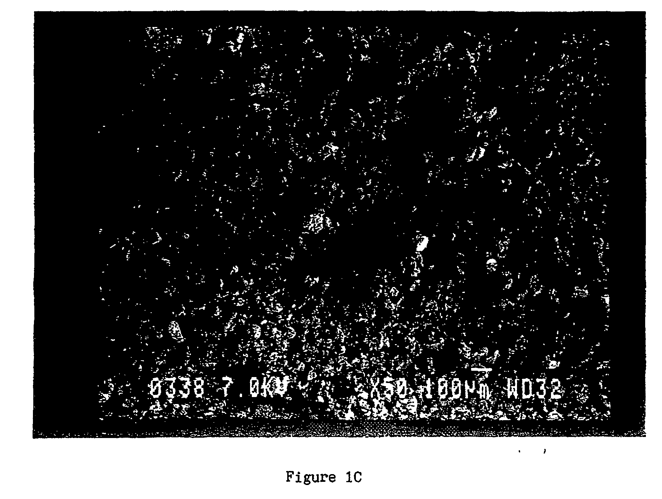

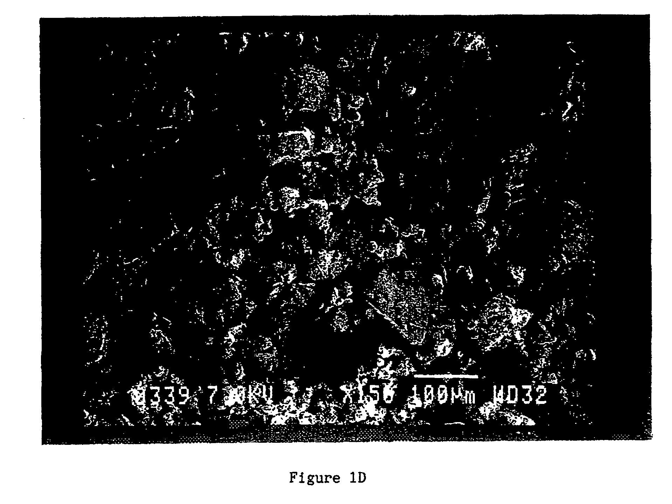

Les cristaux obtenus par la présente invention ont des formes régulières et ne sont pas agglomérés, comme le montre la figure 1. D'une manière générale, et dans les exemples qui suivent, l'ouverture moyenne des cristaux est centrée autour d'une valeur bien déterminée et ceci n'est pas le cas dans les procédés décrits par l'art antérieur. Les exemples suivants illustrent l'invention et ne sont en aucun cas interprétés comme limitant le procédé.

Description des figures :

La figure 1A représente une photographie d'une composition de sucre microcristallin de 80 μm observée au microscope électronique au grossissement X50.

La figure 1B représente une photographie d'une composition de sucre microcristallin de 80 μm observée au microscope électronique au grossissement X150.

La figure 1C représente une photographie d'une composition de sucre glace commercial observée au microscope électronique au grossissement X50.

La figure 1D représente une photographie d'une composition de sucre glace commercial observée au microscope électronique au grossissement X150.

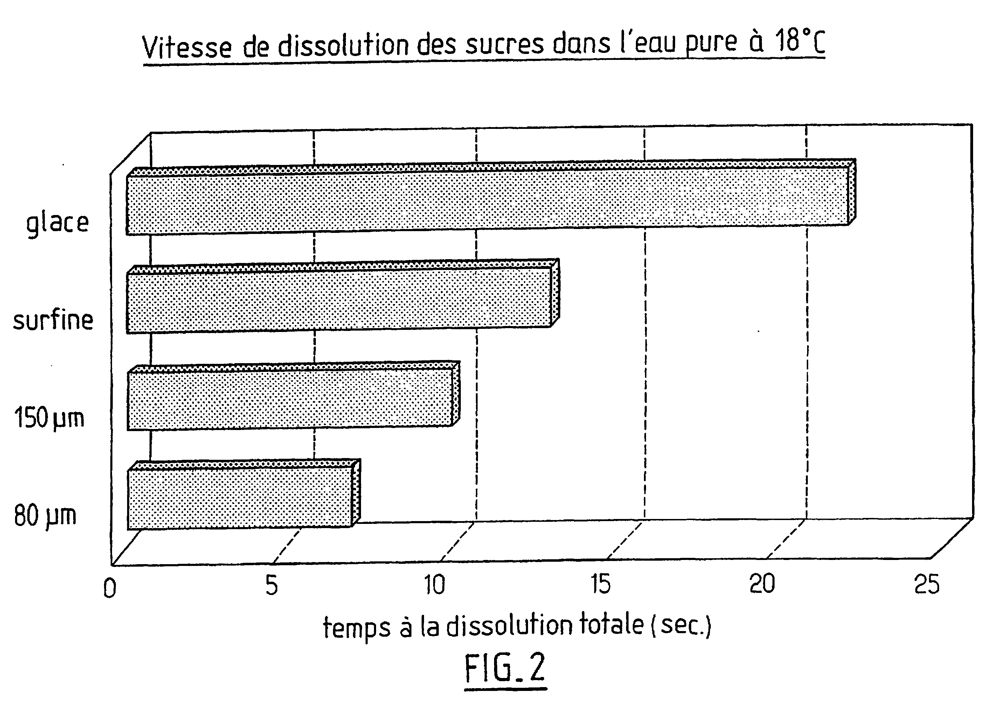

La figure 2 représente la vitesse de dissolution de sucres dans l'eau pure à 18°C. Le temps correspondant à la dissolution totale (exprimé en secondes) est porté sur l'axe des abscisses. Les différents sucres testés sont portés sur l'axe des ordonnées, étant rappelé que le sucre glace a une granulométrie de 80 à 100 μm, que la surfine a une granulométrie de 200 à 250 μm et que les sucres de granulométrie respective de 150 μm et de 80 μm correspondent aux compositions de l'invention.

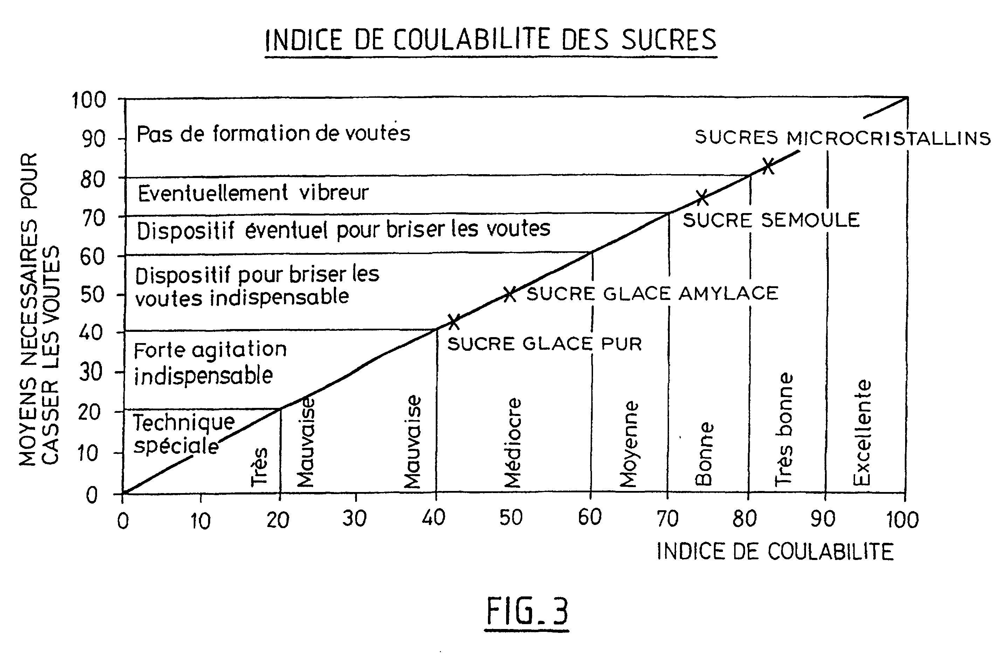

La figure 3 représente l'indice de coulabilité.

Les produits pulvérulents peuvent former des agglomérats dans les réservoirs de stockage et trémies d'alimentation. Le vidage de ces réservoirs et autres trémies est rendu difficile par ce phénomène, entraînant la formation de voûtes (blocs de poudre restant accrochés aux parois des trémies et au-dessus d'une cavité, et formant des zones mortes), perturbant l'écoulement de la poudre par simple gravité. Il est alors nécessaire d'utiliser tout dispositif mécanique permettant de maintenir cette poudre en mélange homogène, en la stockant sous agitation ou en la soutirant de la trémie à l'aide d'écluses rotatives ou de vibreurs.

La difficulté à manipuler un produit pulvérulent est traduite par son indice de coulabilité qui peut varier de 0 (produit à forte capacité d'agglomération, mottant, collant) à 100 (produit extrêmement fluide de comportement proche de celui d'un liquide).

Les faibles indices nécessitent un équipement spécial adapté à chaque cas, les forts indices ne posant pas de problème particulier en stockage et manutention.

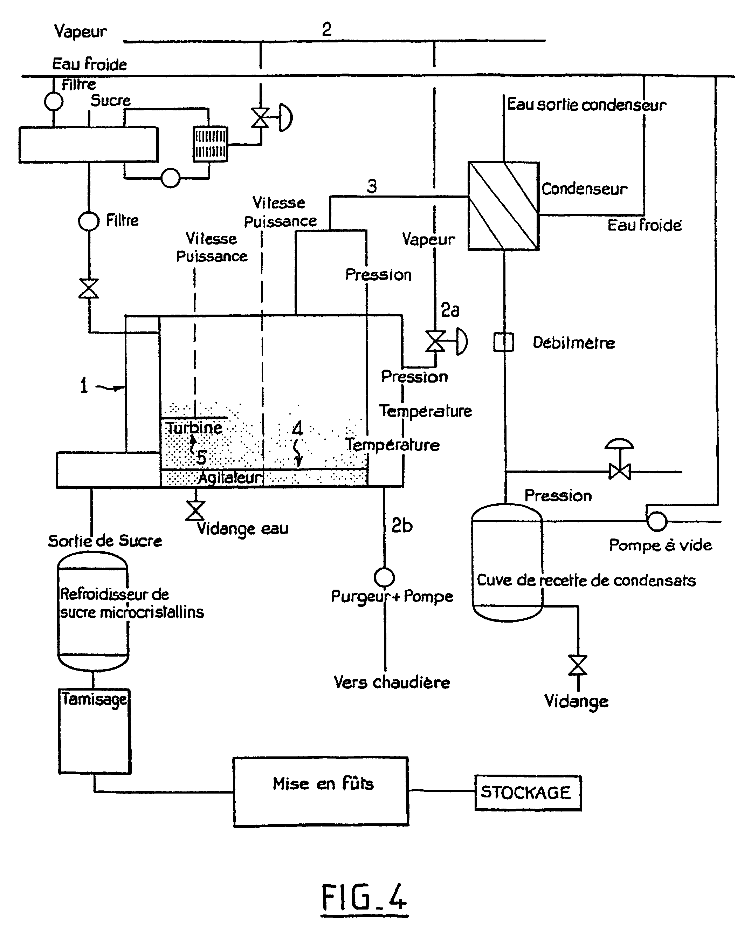

La figure 4 représente un schéma de principe de l'appareillage utilisé dans le cadre des exemples 1 à 7.

L'appareillage utilisé peut être constitué par un évaporateur mélangeur constitué d'une enceinte (1) susceptible de fonctionner sous pression réduite et température régulée. A cette fin, cette enceinte comporte un fluide caloporteur (2), dont la prise d'entrée est par exemple en 2a et la prise de sortie en 2b, et est reliée à une prise de vide (3).

Le mélange (ou brassage) du sirop de sucre au cours du procédé est assuré par un mobile d'agitation (4).

Le battage par impact est effectué par exemple par un couteau émotteur télescopique (5).

Exemple 1 :

Préparation de sucre microcristallin d'ouverture moyenne 80 μm et de CV = 40%.

Vingt kg de sucre sont mis en solution dans 6 kg d'eau à 80°C, température qui sera maintenue constante tout au long de la préparation.

La vitesse de brassage est fixée à 245 m/min (vitesse périphérique). L'évaporation du sirop est conduite jusqu'à une sursaturation d'une valeur comprise entre 1,1 et 1,3 et avantageusement 1,2 et l'action de l'émotteur (environ 1000 tours/mn) est effective dès que la valeur de la sursaturation déterminée est atteinte. Le débit d'évaporation est maintenu à une valeur de 1,5 l/h sous 250 mbars.

Après 15 mn dans ces conditions, un voile blanc significatif de la nucléation apparaît dans le milieu. Cet état est maintenu pendant 40 mn, l'action de l'émotteur permettant de multiplier le nombre de germes en limitant leur croissance.

L'évaporation et le battage sont stoppés pendant 10 minutes. pour laisser la place à une phase de croissance cristalline régulière.

Dans la dernière étape, la vitesse d'agitation du mobile de brassage est fixée à 190 m/mn (vitesse périphérique) et l'évaporation est poursuivie avec un débit croissant jusqu'à l'obtention d'une poudre sèche.

Durée globale de l'opération : 3 heures.

La composition de l'invention ainsi obtenue a les propriétés suivantes :

vitesse de dissolution : 7 sec.
indice de coulabilité : 81
densité de la composition tassée : 0,97
densité de la composition non tassée : 0,83.

Exemple 2 :

Préparation de sucre microcristallin d'ouverture moyenne 150 µm et de CV = 30%.

Vingt kg de sucre sont dissous dans 6 kg d'eau à 80°C, température qui sera maintenue constante tout au long de l'opération.
La vitesse d'agitation du mobile de brassage est fixée à 135 m/mn (vitesse périphérique). L'évaporation du sirop est conduite jusqu'à une sursaturation d'une valeur comprise entre 1,1 et 1,3 et avantageusement 1,2 et l'action de l'émotteur (environ 1000 tours/mn) est effective dès que la valeur de la sursaturation déterminée est atteinte.
Le débit d'évaporation est maintenu à une valeur de 1,5 l/h sous 250 mbars.
Après 10 mn dans ces conditions, un voile blanc significatif de la nucléation apparaît dans le milieu. Cet état est maintenu pendant 5 mn, puis l'évaporation et l'émotteur sont stoppés pendant 15 minutes, favorisant la phase de croissance cristalline.
Dans la deuxième étape, la vitesse d'agitation du mobile de brassage est fixée à 135 m/mn (vitesse périphérique) et l'évaporation est poursuivie avec un débit croissant jusqu'à l'obtention d'une poudre sèche.
Durée globale de l'opération : 5 heures.
La composition de l'invention ainsi obtenue a les propriétés suivantes :

vitesse de dissolution : 9 sec.
indice de coulabilité : 82
densité de la composition tassée : 1,00
densité de la composition non tassée : 0,87.

Exemple 3 :

Préparation de sucre microcristallin d'ouverture moyenne 150 µm et de CV = 30% contenant du caramel.

Vingt kg de sucre sont dissous dans 6 kg d'eau à 80°C, température qui sera maintenue constante tout au long de l'opération.
La vitesse d'agitation du mobile de brassage est fixée à 135 m/mn (vitesse périphérique). L'évaporation du sirop est conduite jusqu'à une sursaturation d'une valeur comprise entre 1,1 et 1,3 et avantageusement 1,2 et l'action de l'émotteur (environ 1000 tours/mn) est effective dès que la valeur de la sursaturation déterminée est atteinte.
Le débit d'évaporation est maintenu à une valeur de 1,5 l/h sous 250 mbars.
A cet instant, 400 g de caramel aromatique représentant 2% de la masse totale de sucre sont dilués dans le milieu.
Après 5 minutes d'homogénéisation, l'évaporation et l'émotteur sont stoppés pendant 15 minutes, favorisant la phase de croissance cristalline.
Dans la dernière étape, la vitesse d'agitation du mobile de brassage est fixée à 135 m/mn (vitesse périphérique) et l'évaporation est poursuivie avec un débit croissant jusqu'à l'obtention d'une poudre sèche.
Durée globale de l'opération : 5 heures.
La composition de l'invention ainsi obtenue a les mêmes caractéristiques que la composition obtenue à l'exemple 2.

Exemple 4 :

Préparation de glucose microcristallin d'ouverture moyenne 75 µm.

Dix huit kg de glucose sont dissous dans 5,4 kg d'eau à 70°C. température qui sera maintenue constante tout au long de l'opération.
La vitesse d'agitation du mobile de brassage est fixée à 245 m/mn (vitesse périphérique).
L'évaporation du sirop est conduite jusqu'à une sursaturation d'une valeur comprise entre 1,1 et 1,4 et avantageusement de 1,3 et l'action de l'émotteur (environ 1000 tours/mn) est effective dès que la valeur de la sursaturation déterminée est atteinte. Le débit d'évaporation est maintenu à une valeur moyenne de 1,5 l/h sous environ 180 mbars.

Après 100 mn dans ces conditions, un voile blanc significatif de la nucléation apparaît dans le milieu. Cet état est maintenu pendant 40 mn, l'action de l'émotteur permettant de multiplier le nombre de germes en limitant la croissance.

L'évaporation et le battage sont stoppés pendant 10 minutes, favorisant la phase de croissance cristalline.

Dans la deuxième étape, la vitesse d'agitation du mobile est fixée à 140 m/mn (vitesse périphérique) et l'évaporation est poursuivie avec un débit croissant jusqu'à obtention d'une poudre sèche.

Durée globale de l'opération : 4 heures.

La composition de l'invention ainsi obtenue a les propriétés suivantes :

- indice de coulabilité : 60
- densité de la composition tassée : 0,75
- densité de la composition non tassée : 0,52.

Exemple 5 :

Préparation de lactose microcristallin d'ouverture moyenne 50 μm.

Quinze kg de lactose sont dissous dans 20 kg d'eau à 72°C, température qui sera maintenue constante tout au long de l'opération.

La vitesse d'agitation du mobile de brassage est fixée à 245 m/mn (vitesse périphérique).

L'évaporation du sirop est conduite jusqu'à une sursaturation d'une valeur comprise entre 1,1 et 1,3 et avantageusement de 1,1 et l'action de l'émotteur (environ 1000 tours/mn) est effective dès que la valeur de la sursaturation déterminée est atteinte. Le débit d'évaporation est maintenu à une valeur moyenne de 2,5 l/h sous environ 180 mbars.

Après 220 mn dans ces conditions, un voile blanc significatif de la nucléation apparaît dans le milieu. Ce état est maintenu pendant 40 mn, l'action de l'émotteur permettant de multiplier le nombre de germes en limitant la croissance.

L'évaporation et le battage sont stoppés pendant 10 minutes, favorisant la phase de croissance cristalline.

Dans la deuxième étape, la vitesse d'agitation du mobile est fixée à 140 m/mn (vitesse périphérique) et l'évaporation est poursuivie avec un débit croissant jusqu'à obtention d'une poudre sèche.

Durée globale de l'opération : 7 heures.

La composition de l'invention ainsi obtenue a les propriétés suivantes :

- vitesse de dissolution : non soluble dans les conditions de l'essai
- indice de coulabilité : 80
- densité de la composition tassée : 0,93
- densité de la composition non tassée : 0,83.

Exemple 6 :

Préparation d'érythritol microcristallin d'ouverture moyenne 220 μm.

Dix huit kg d'érythritol sont dissous dans 8 kg d'eau à 70°C, température qui sera maintenue constante tout au long de l'opération.

La vitesse d'agitation du mobile de brassage est fixée à 245 m/mn (vitesse périphérique).

L'évaporation du sirop est conduite jusqu'à une sursaturation d'une valeur comprise entre 1,1 et 1,3 et avantageusement de 1,1 et l'action de l'émotteur (environ 1000 tours/mn) est effective dès que la valeur de la sursaturation déterminée est atteinte. Le débit d'évaporation est maintenu à une valeur moyenne de 2,0 l/h sous environ 180 mbars.

Après 40 mn dans ces conditions, un voile blanc significatif de la nucléation apparaît dans le milieu.

L'évaporation et le battage sont stoppés pendant 10 minutes, favorisant la phase de croissance cristalline.

Dans la deuxième étape, la vitesse d'agitation du mobile est fixée à 140 m/mn (vitesse périphérique) et l'évaporation est poursuivie avec un débit croissant jusqu'à obtention d'une poudre sèche.

Durée globale de l'opération : 3,5 heures.

La composition de l'invention ainsi obtenue a les propriétés suivantes :

- vitesse de dissolution : non soluble dans les conditions de l'essai (trouble persistant)
- indice de coulabilité : 83
- densité de la composition tassée : 0,92
- densité de la composition non tassée : 0,90.

Exemple 7 :

Essai industriel.

Préparation de saccharose microcristallin d'ouverture moyenne 120 µm.

Dans un mélangeur Guedu de 1600 litres, 800 kg de saccharose sont dissous dans 310 kg d'eau à 62°C, température qui sera maintenue constante tout au long de l'opération.

La vitesse d'agitation du mobile de brassage est fixée à 330 m/mn (vitesse périphérique).

L'action de l'émotteur (environ 1000 tours/mn) est effective dès le début de l'évaporation et tout au long de l'opération. La concentration du sirop est conduite avec un débit d'évaporation moyen de 20 à 30%/heure. L'énergie apportée au système (chauffage vapeur, double enveloppe) est régulée par la consigne de débit prédéterminé.

L'évaporation est effectuée sous pression réduite, pression définie par la température du sirop pour obtenir l'ébullition du milieu à cette température.

Le système est maintenu dans cet état d'équilibre, débit d'évaporation/pression/température jusqu'à un taux d'évaporation de 65% environ.

Le milieu devient alors très pâteux et, dans cette deuxième étape, la vitesse d'agitation du mobile est abaissée à 190 m/mn (vitesse périphérique) et l'évaporation est poursuivie avec une pression décroissant progressivement pour maintenir une température constante jusqu'à obtention d'une poudre sèche.

En fin de cycle, le produit est déchargé sans refroidissement préalable, les grugeons éventuellement présents dans la poudre sont éliminés par passage rapide sur un tamis de 300 µm. Les cristaux obtenus ne mottent pas après plusieurs jours de stockage à l'air ambiant.

Durée globale de l'opération : 6 heures.

La composition de l'invention ainsi obtenue a les propriétés suivantes:

- vitesse de dissolution : 8 sec.
- indice de coulabilité ; 82
- densité de la composition tassée : 0,98
- densité de la composition non tassée : 0,84.

Exemple comparatif :

| | Sucre glace | Semoule surfine | Exemple 1 saccharose | Exemple 2 saccharose | Exemple 3 saccharose | Exemple 4 glucose | Exemple 5 lactose | Exemple 6 érythritol | Exemple 7 saccharose |
|---|---|---|---|---|---|---|---|---|---|
| OM ($\mu$m) | < 80 | 250 | 80 | 150 | 150 | 75 | 50 | 220 | 120 |
| C.V. (%) | N.D.* | N.D.* | 35-45 | 30-40 | 30-40 | N.D.* | N.D.* | N.D.* | 50 |
| Indice de coulabilité | 42 | 75 | 81 | 82 | 82 | 60 | 80 | 83 | 82 |
| Vitesse de dissolution (sec.) | 22** | 13 | 7 | 9 | 9 | N.D.* | N.D.* | N.D.* | 8 |
| Densité produit non tassé | 0,45 | 0,65 | 0,83 | 0,87 | 0,87 | 0,52 | 0,83 | 0,90 | 0,84 |
| Densité produit tassé | 0,88 | 0,87 | 0,97 | 1,00 | 1,00 | 0,75 | 0,93 | 0,92 | 0,98 |
| Mottage | oui | non motté | non motté | non motté | non motté | non motté | non motté | non motté | non motté |

* N.D. : non déterminé

** : problème de mouillabilité

EP 0 870 064 B1

**Revendications**

1.  Composition contenant des microcristaux de sucre **caractérisée en ce que** les cristaux sont essentiellement des monocristaux de forme géométrique régulière, ne présentant pas de brisure, homogènes les uns par rapport aux autres, et **en ce que**

    -   la granulométrie suit une distribution gaussienne dont la médiane est d'environ 20 μm à environ 220 μm, notamment d'environ 20 μm à environ 200 μm, le coefficient de variation étant d'environ 20% à environ 50%, notamment d'environ 30% à 45%, ou d'environ 35% à 45%, ou d'environ 30% à 40% ou
    -   la répartition granulométrique est **caractérisée par** un indice d'uniformité compris entre 1 et 5, notamment entre 2.5 et 3.5.

2.  Composition selon la revendication 1, **caractérisée en ce qu'**elle présente les propriétés suivantes :

    -   sa vitesse de dissolution est d'environ 5 à environ 10, notamment d'environ 7 à environ 9 secondes, dans les conditions suivantes : 10 g de composition pour 100 ml d'eau pure déminéralisée, à la température de 18°C,
    -   elle est non mouante,
    -   son indice de coulabilité est supérieur à environ 80, et varie d'environ 80 à environ 85, notamment d'environ 81 à environ 82, mesuré selon le test d'Hosakawa, tel que décrit dans IRON WORKS, LTD, Osaka, Japon, et lorsqu'il s'agit du glucose, l'indice de coulabilité est d'environ 55 à environ 70,
    -   la densité du produit tassé est d'environ 0,90 à environ 1,00, notamment d'environ 0,97 à environ 1,00, et la densité du produit non tassé est d'environ 0,75 à environ 0,90, notamment d'environ 0,83 à environ 0,87, mesurées selon le test d'Hosakawa, et lorsque le susdit produit est du glucose, la densité du produit tassé est d'environ 0,70 à environ 0,90 et la densité du produit non tassé est d'environ 0,50 à environ 0,70.

3.  Composition selon l'une des revendications 1 ou 2, **caractérisée en ce qu'**elle contient des ingrédients additionnels, à raison d'environ 0% à environ 10%, et avantageusement à raison d'environ 5%, ces ingrédients étant de façon avantageuse choisis parmi les composés thermosensibles, des composés ayant des propriétés alimentaires ou pharmacologiques, ou des composés ayant un goût ou une couleur recherchés.

4.  Composition selon l'une des revendications 1 à 3, susceptible d'être obtenue par le procédé comprenant les étapes suivantes :

    a) on prépare un sirop de saccharose concentré, d'environ 60 à environ 97, notamment 75% en poids de matières sèches,
    b) on réduit la pression qui passe de la pression atmosphérique à une valeur d'environ 100 à environ 300 mbars, notamment d'environ 200 mbars, pour commencer à évaporer une partie de l'eau contenue dans le sirop de sucre, le taux d'évaporation étant d'environ 20%,
    c) on évapore une partie de l'eau contenue dans le sirop de sucre sous pression réduite (environ 200 mbars) et on brasse le sirop, notamment par agitation mécanique à une vitesse périphérique d'environ 100 à environ 350 m/mn, jusqu'à atteindre un coefficient de sursaturation de sucre compris entre 1,1 et 1,3 et on provoque la cristallisation par une agitation vigoureuse du sirop (en plus du brassage susmentionné), notamment par chocs mécaniques générés par battage par impact, dans cette zone de sursaturation,
    d) on effectue la suite de la cristallisation par arrêt de l'évaporation et de l'agitation vigoureuse (battage), et maintien d'une agitation régulière (brassage) pendant le temps nécessaire à l'obtention des cristaux de taille souhaitée, et avantageusement pendant environ 5 mn à environ 20 mn,
    e) on reprend l'évaporation (toujours avec brassage du milieu à une vitesse d'environ 100 à environ 350 m/mn) jusqu'à l'obtention de cristaux contenant moins de 1%, notamment moins de 0,5% d'humidité,

    la température étant maintenue à une valeur d'environ 70°C à environ 100°C, pendant toute la durée du procédé, et la pression étant avantageusement maintenue à environ 200 mbars pendant les étapes c) à e).

5.  Composition selon l'une quelconque des revendications 1 à 3, susceptible d'être obtenue par le procédé comprenant les étapes suivantes :

    a) on prépare un sirop de sucre concentré d'environ 60% à environ 97%, notamment 75% en poids de matières sèches,
    b) on provoque l'évaporation du sirop par réduction de la pression de manière à atteindre l'ébullition de ce sirop à la température choisie,

c) on brasse le sirop notamment par agitation mécanique à une vitesse périphérique d'environ 100 à environ 350 m/mn, notamment 200 à 350 m/mn, le coefficient de sursaturation du sirop étant compris entre 1 et 1,3, notamment 1,1 et 1,3, et on provoque la cristallisation par une agitation vigoureuse du sirop (en plus du brassage susmentionné), notamment par chocs mécaniques générés par battage par impact, dans cette zone de sursaturation,

d) on poursuit l'évaporation dans les mêmes conditions de température et de pression que celles utilisées dans les étapes précédentes, jusqu'à obtention d'un milieu dont les cristaux constituent la phase majoritaire (supérieur à environ 50%, et notamment supérieur à environ 70% par rapport au milieu),

la vitesse d'agitation étant réduite d'environ 50 à environ 200 m/mn, la température étant maintenue constante par rapport aux étapes précédentes, le battage étant maintenu jusqu'à obtention d'un produit sec composé de cristaux de taille souhaitée contenant moins de 1%, notamment moins de 0,5% d'humidité,

la température étant ajustée et maintenue à une valeur constante dans la gamme allant d'environ 40°C à environ 100°C, notamment d'environ 70°C à environ 100°C, pendant toute la durée des étapes.

**6.** Procédé de préparation d'une composition selon l'une quelconque des revendications 1 à 4, **caractérisé par** les étapes suivantes :

a) on prépare un sirop de saccharose concentré, d'environ 60 à environ 97, notamment 75% en poids de matières sèches,

b) on réduit la pression qui passe de la pression atmosphérique à une valeur d'environ 100 à environ 300 mbars, notamment d'environ 200 mbars, pour commencer à évaporer une partie de l'eau contenue dans le sirop de sucre, le taux d'évaporation étant d'environ 20%,

c) on évapore une partie de l'eau contenue dans le sirop de sucre sous pression réduite (environ 200 mbars) et on brasse le sirop, notamment par agitation mécanique à une vitesse périphérique d'environ 100 à environ 350 m/mn, jusqu'à atteindre un coefficient de sursaturation de sucre compris entre 1,1 et 1,3 et on provoque la cristallisation par une agitation vigoureuse du sirop (en plus du brassage susmentionné), notamment par chocs mécaniques générés par battage par impact, dans cette zone de sursaturation,

d) on effectue la suite de la cristallisation par arrêt de l'évaporation et de l'agitation vigoureuse (battage), et maintien d'une agitation régulière (brassage) pendant le temps nécessaire à l'obtention des cristaux de taille souhaitée, et avantageusement pendant environ 5 mn à environ 20 mn,

e) on reprend l'évaporation (toujours avec brassage du milieu à une vitesse d'environ 100 à environ 350 m/mn) jusqu'à l'obtention de cristaux contenant moins de 1%, notamment moins de 0,5% d'humidité,

la température étant maintenue à une valeur d'environ 70°C à environ 100°C, pendant toute la durée du procédé, et la pression étant avantageusement maintenue à environ 200 mbars pendant les étapes c) à e).

**7.** Procédé de préparation d'une composition selon l'une quelconque des revendications 1 à 3 et 5, **caractérisé par** les étapes suivantes :

a) on prépare un sirop de sucre concentré d'environ 60% à environ 97%, notamment 75% en poids de matières sèches,

b) on provoque l'évaporation du sirop par réduction de la pression de manière à atteindre l'ébullition de ce sirop à la température choisie,

c) on brasse le sirop notamment par agitation mécanique à une vitesse périphérique d'environ 100 à environ 350 m/mn, notamment 200 à 350 m/mn, le coefficient de sursaturation du sirop étant compris entre 1 et 1,3, notamment 1,1 et 1,3, et on provoque la cristallisation par une agitation vigoureuse du sirop (en plus du brassage susmentionné), notamment par chocs mécaniques générés par battage par impact, dans cette zone de sursaturation,

d) on poursuit l'évaporation dans les mêmes conditions de température et de pression que celles utilisées dans les étapes précédentes, jusqu'à obtention d'un milieu dont les cristaux constituent la phase majoritaire (supérieur à environ 50%, et notamment supérieur à environ 70% par rapport au milieu),

la vitesse d'agitation étant réduite d'environ 50 à environ 200 m/mn, la température étant maintenue constante par rapport aux étapes précédentes, le battage étant maintenu jusqu'à obtention d'un produit sec composé de cristaux de taille souhaitée contenant moins de 1%, notamment moins de 0,5% d'humidité,

la température étant ajustée et maintenue à une valeur constante dans la gamme allant d'environ 40°C à environ

**EP 0 870 064 B1**

100°C, notamment d'environ 70°C à environ 100°C, pendant toute la durée des étapes.

**Claims**

1. A composition containing sugar microcrystals, **characterised in that** the crystals are essentially unbroken monocrystals with a regular geometric shape, homogeneous with respect to one another, and **in that**

   - the particle size follows a Gaussian distribution of which the median is about 20 μm to about 220 μm, particularly about 20 μm to about 200 μm, the coefficient of variation being about 20% to about 50%, particularly about 30% to 45% or about 35% to 45%, or about 30% to 40%, or
   - the particle size distribution is **characterised by** a uniformity index between 1 and 5, particularly between 2.5 and 3.5.

2. A composition according to claim 1, **characterised in that** it has the following properties:

   - its rate of dissolution is about 5 to about 10, particularly about 7 to about 9 seconds, under the following conditions: 10 g of composition per 100 ml of pure demineralised water at a temperature of 18°C,
   - it is non caking,
   - its pourability index is greater than about 80, and ranges from about 80 to about 85, particularly about 81 to about 82, measured according to the Hosakawa test as described in IRON WORKS LTD, Osaka, Japan, and if it is glucose, the pourability index is about 55 to about 70,
   - the specific gravity of the compacted product is about 0.90 to about 1.00, particularly about 0.97 to about 1.00, and the specific gravity of the uncompacted product is about 0.75 to about 0.90, particularly about 0.83 to about 0.87, measured according to the Hosakawa test, and if the said product is glucose, the specific gravity of the compacted product is about 0.70 to about 0.90 and the specific gravity of the uncompacted product is about 0.50 to about 0.70.

3. A composition according to one of claims 1 or 2, **characterised in that** it contains additional ingredients in a quantity of about 0% to about 10%, and advantageously in a quantity of about 5%, these ingredients being chosen advantageously from heat-sensitive compounds, compounds having food or pharmacological properties or compounds having a desired taste or colour.

4. A composition according to one of claims 1 to 3, which may be obtained by the process comprising the following steps:

   a) a concentrated sucrose syrup is prepared containing about 60 to about 97, particularly 75% by weight of dry matter,
   b) the pressure is reduced from atmospheric pressure to a value of about 100 to about 300 mbars, particularly about 200 mbars in order to start evaporating part of the water contained in the sugar syrup, the rate of evaporation being about 20%,
   c) a part of the water contained in the sugar syrup is evaporated under reduced pressure (about 200 mbars) and the syrup is stirred, particularly by mechanical agitation at a peripheral speed of about 100 to about 350 m/mn until a coefficient of supersaturation of sugar between 1.1 and 1.3, and crystallisation is brought about by vigorous agitation of the syrup (in addition to the stirring mentioned above), particularly by mechanical shocks generated by beating by impact, in this supersaturation zone,
   d) crystallisation is continued by stopping evaporation and vigorous agitation (beating) and maintaining regular agitation (stirring) for the time required to obtain crystals of the desired size, and advantageously for about 5 min to about 20 min,
   e) evaporation is resumed (still whilst stirring the medium at a speed of about 100 to about 350 m/min) until crystals containing less than 1% and particularly less than 0.5% of moisture are obtained,

   the temperature being kept at a value of about 70°C to about 100°C throughout the process, and the pressure being advantageously kept at about 200 mbars during steps c) to e).

5. A composition according to any one of claims 1 to 3, which may be obtained by the process comprising the following steps:

**14**

a) a concentrated sugar syrup is prepared containing about 60% to about 97%, particularly 75% by weight of dry matter,

b) evaporation of the syrup is brought about by reducing the pressure so as to achieve boiling of this syrup at the chosen temperature,

c) the syrup is stirred particularly by mechanical agitation at a peripheral speed of about 100 to about 350 m/min, particularly 200 to 350 m/min, the coefficient of supersaturation of the syrup being between 1 and 1.3, particularly 1.1 and 1.3, and crystallisation is brought about by vigorous agitation of the syrup (in addition to the stirring mentioned above), particularly by mechanical shocks generated by beating by impact, in this supersaturation zone,

d) evaporation is continued under the same temperature and pressure conditions as those used in the previous steps, until a medium is obtained in which the crystals constitute the majority phase (more than about 50%, and particularly more than about 70% with respect to the medium),

the rate of agitation being reduced by about 50 to about 200 m/min, the temperature being kept constant with respect to the previous steps, beating being maintained until a dry product is obtained composed of crystals of the desired size containing less than 1%, particularly less than 0.5% of moisture,

the temperature being adjusted and kept at a constant value within the range from about 40°C to about 100°C, particularly about 70°C to about 100°C throughout the duration of the steps.

6. A process for the preparation of a composition according to any one of claims 1 to 4, **characterised by** the following steps:

a) a concentrated sucrose syrup is prepared containing about 60 to about 97, particularly 75% by weight of dry matter,

b) the pressure is reduced from atmospheric pressure to a value of about 100 to about 300 mbars, particularly about 200 mbars in order to start evaporating a part of the water contained in the sugar syrup, the rate of evaporation being about 20%.

c) a part of the water contained in the sugar syrup is evaporated under reduced pressure (about 200 mbars) and the syrup is stirred particularly by mechanical agitation at a peripheral speed of about 100 to about 350 m/min until a coefficient of supersaturation of sugar between 1.1 and 1.3 is obtained, and crystallisation is brought about by vigorous agitation of the syrup (in addition to the stirring mentioned above), particularly by mechanical shocks generated by beating by impact, in this supersaturation zone,

d) crystallisation is continued by stopping evaporation and vigorous agitation (beating) and maintaining regular agitation (stirring) for the time required to obtain crystals of the desired size, and advantageously for about 5 min to about 20 min,

e) evaporation is resumed (still whilst stirring the medium at a rate of about 100 to about 350 m/min), until crystals containing less than 1%, particularly less than 0.5% of moisture are obtained,

the temperature being kept at a value of about 70°C to about 100°C throughout the process, and the pressure being advantageously kept at about 200 mbars during steps c) to e).

7. A process for the preparation of a composition according to any one of claims 1 to 3 and 5, **characterised by** the following steps:

a) a concentrated sugar syrup is prepared containing about 60% to about 97%, particularly 75% by weight of dry matter,

b) evaporation of the syrup is brought about by reducing the pressure so as to achieve boiling of this syrup at the chosen temperature,

c) the syrup is stirred particularly by mechanical agitation at a peripheral speed of about 100 to about 350 m/min, particularly 200 to 350 m/min, the coefficient of supersaturation of the syrup being between 1 and 1.3, particularly 1.1 and 1.3, and crystallisation is brought about by vigorous agitation of the syrup (in addition to the stirring mentioned above), particularly by mechanical shocks generated by beating by impact, in this supersaturation zone,

d) evaporation is continued under the same temperature and pressure conditions as those used in the previous steps, until a medium is obtained in which the crystals constitute the majority phase (more than about 50%, and particularly more than about 70% with respect to the medium),

the rate of agitation being reduced by about 50 to about 200 m/min, the temperature being kept constant with respect to the previous steps, beating being maintained until a dry product is obtained composed of crystals of the desired size containing less than 1%, particularly less than 0.5% of moisture,
the temperature being adjusted and kept at a constant value within the range from about 40°C to about 100°C, particularly about 70°C to about 100°C throughout the duration of the steps.

**Patentansprüche**

1. Zusammensetzung, die Mikrokristalle von Zucker enthält, **dadurch gekennzeichnet, dass** die Kristalle im Wesentlichen Monokristalle mit regelmäßiger Geometrie sind, keinen Bruch aufweisen, wobei die einen in Bezug zu den anderen homogen sind, und, dass

   - die Korngrößenverteilung eine Gauss-Verteilung annimmt, von der der Median von ungefähr 20 µm bis ungefähr 220 µm ist, insbesondere von ungefähr 20 µm bis ungefähr 200 um, wobei der Abweichungskoeffizient ungefähr 20% bis ungefähr 50% beträgt, insbesondere von ungefähr 30% bis 45%, oder von ungefähr 35% bis 45%, oder von ungefähr 30% bis 40% oder
   - die Korngrößenverteilung durch einen Gleichförmigkeitsindex **gekennzeichnet** ist, der zwischen 1 und 5, insbesondere zwischen 2,5 und 3,5 umfasst.

2. Zusammensetzung gemäß Anspruch 1, **dadurch gekennzeichnet, dass** sie die folgenden Eigenschaften besitzt:

   - ihre Auflösungsgeschwindigkeit von ungefähr 5 bis ungefähr 10, insbesondere von ungefähr 7 bis ungefähr 9 Sekunden, unter den folgenden Bedingungen beträgt: 10 g Zusammensetzung auf 100 ml reines demineralisiertes Wasser bei einer Temperatur von 18°C,
   - sie nicht verklumpend ist,
   - ihr Fließfähigkeitsindex oberhalb von ungefähr 80 liegt und von ungefähr 80 bis ungefähr 85, insbesondere von ungefähr 81 bis ungefähr 82 variiert, gemessen gemäß dem Hosakawa-Test, sowie in IRON WORKS, Ltd., Osaka, Japan beschrieben, und, wenn es sich um Glucose handelt, der Fließfähigkeitsindex von ungefähr 55 bis ungefähr 70 beträgt,
   - die Dichte des tassierten Produktes von ungefähr 0,90 bis ungefähr 1,00, insbesondere von ungefähr 0,97 bis ungefähr 1,00 beträgt, und die Dichte des nicht tassierten Produktes von ungefähr 0,75 bis 0,90, insbesondere von ungefähr 0,83 bis ungefähr 0,87 beträgt, gemessen gemäß dem Hosakawa-Test, und, wenn das vorgenannte Produkt Glucose ist, die Dichte des tassierten Produktes von ungefähr 0,70 bis ungefähr 0,90 beträgt und die Dichte des nicht tassierten Produktes von ungefähr 0,50 bis ungefähr 0,70 beträgt.

3. Zusammensetzung gemäß einem der Ansprüche 1 oder 2, **dadurch gekennzeichnet, dass** sie zusätzliche Inhaltsstoffe in einer Menge von 0% bis ungefähr 10%, und vorzugsweise in einer Menge von ungefähr 5% enthält, wobei diese Inhaltsstoffe vorzugsweise unter thermisch empfindlichen Verbindungen ausgewählt sind, wobei die Verbindungen Lebensmittel- oder pharmakologische Eigenschaften aurweisen oder die Verbindungen einen Geschmack oder eine Farbe aufweisen, die ausgesucht sind.

4. Zusammensetzung gemäß einem der Ansprüche 1 bis 3, erhältlich gemäß dem Verfahren, das folgende Schritte umfasst:

   a) Herstellen eines konzentrierten Saccharose-Sirups, von ungefähr 60 bis ungefähr 97, insbesondere 75 Gew.-% der Trockenmasse,
   b) Reduzieren des Drucks von atmosphärischen Druck bis auf einen Wert von ungefähr 100 bis ungefähr 300 mbar, insbesondere auf ungefähr 200 mbar, um mit dem Verdampfen eines Teils des Wassers, das in dem Zuckersirup enthalten ist, anzufangen,
   wobei die Menge der Verdampfung ungefähr 20% beträgt,
   c) Verdampfen eines Teils des in dem Zuckersirup enthaltenen Wassers unter reduziertem Druck (ungefähr 200 mbar) und Mischen des Sirups, insbesondere durch mechanische Bewegung bei einer peripheren Geschwindigkeit von ungefähr 100 bis ungefähr 350 U/min, bis zum Erreichen eines Übersättigungskoeffizienten des Zuckers, der zwischen 1,1 und 1,3 umfasst, und Provozieren der Kristallisation durch eine heftige Bewegung des Sirups (zusätzlich zu dem vorstehend erwähnten Mischen), insbesondere durch durch Stoßeinschlag erzeugte mechanische Schocks, in dieser Zone der Übersättigung,
   d) Bewirken des Voranschreitens der Kristallisation durch Anhalten der Verdampfung und der heftigen Bewegung (Schlagen), und Beibehalten einer regelmäßigen Bewegung (Mischen) während der Zeit, die notwendig zum Erhalt von Kristallen mit gewünschter Größe ist und vorzugsweise während ungefähr 5 Minuten bis ungefähr 20 Minuten,

e) Wiederaufnehmen der Verdampfung (immer mit Mischen des Milieus mit einer Geschwindigkeit von ungefähr 100 bis ungefähr 350·U/min) bis zum Erhalt von Kristallen, die weniger als 1%, insbesondere weniger als 0,5% Feuchtigkeit enthalten,

wobei die Temperatur bei einem Wert von ungefähr 70°C bis ungefähr 100°C gehalten wird, während der ganzen Zeit des Verfahrens und der Druck vorzugsweise bei ungefähr 200 mbar während der Schritte c) bis e) gehalten wird.

5. Zusammensetzung gemäß einem der Ansprüche 1 bis 3, erhältlich durch das die folgenden Schritte umfassende Verfahren:

a) Herstellen eines konzentrierten Zuckersirups von ungefähr 60% bis ungefähr 97%, insbesondere 75%, bezogen auf das Gewicht der Trockenmasse,

b) Provozieren der Verdampfung des Sirups durch Verminderung des Drucks, um so das Sieden des Sirups bei der ausgewählten Temperatur zu erreichen,

c) Mischen des Sirups insbesondere durch mechanische Bewegung bei einer peripheren Geschwindigkeit von ungefähr 100 bis ungefähr 350 U/min, insbesondere 200 bis 350 U/min, wobei der Übersättigungskoeffizient des Sirups zwischen 1 und 1,3, insbesondere 1,1 und 1,3 umfasst, und Provozieren der Kristallisation durch eine heftige Bewegung des Sirups (zusätzlich zu dem vorstehend erwähnten Mischen), insbesondere durch durch Schlagstoß erzeugte mechanische Stöße, in der Zone der Übersättigung,

d) Fortfahren mit der Verdampfung unter den gleichen Bedingungen der Temperatur und des Drucks wie diejenigen, die in den vorstehenden Schritten verwendet werden, bis zum Erhalt eines Milieus, bei dem die Kristalle die Hauptphase zusammensetzen (mehr als ungefähr 50%, und insbesondere mehr als ungefähr 70% in Bezug zum Milieu), wobei die Bewegungsgeschwindigkeit von ungefähr 50 bis ungefähr 200 U/min vermindert wird, die Temperatur in Bezug auf die vorhergehenden Schritte konstant gehalten wird, das Schlagen bis zum Erhalt eines trockenen Produktes fortgesetzt wird, das aus Kristallen mit einer gewünschten Größe zusammengesetzt ist, die weniger als 1%, insbesondere weniger als 0,5% Feuchtigkeit enthalten,

die Temperatur auf einen konstanten Wert im Bereich von ungefähr 40°C bis ungefähr 100°C, insbesondere von ungefähr 70°C bis ungefähr 100°C während der Dauer der Schritte eingestellt und gehalten wird.

6. Verfahren zur Herstellung einer Zusammensetzung gemäß einem der Ansprüche 1 bis 4, das durch die folgenden Schritte gekermzeichnet ist:

a) Herstellen eines konzentrierten Saccharose-Sirups von ungefähr 60% bis ungefähr 97%, insbesondere 75 Gew.-%, bezogen auf das Gewicht der Trockenmasse,

b) Vermindern des Drucks von atmosphärischem Druck bis auf einen Wert von ungefähr 100 bis ungefähr 300 mbar, insbesondere ungefähr 200 mbar, um mit der Verdampfung eines Teils des Wassers, das in dem Zuckersirup enthalten ist, anzufangen, wobei die Verdampfungsmenge ungefähr 20% beträgt,

c) Verdampfen eines Teils des Wassers, das in dem Zuckersirup enthalten ist, unter vermindertem Druck (ungefähr 200 mbar) und Mischen des Sirups, insbesondere durch mechanische Bewegung mit einer peripheren Geschwindigkeit von ungefähr 100 bis ungefähr 350 U/min, bis zum Erreichen eines Übersättigungskoeffizienten des Zukkers, der zwischen 1,1 und 1,3 umfasst und Provozieren der Kristallisation durch heftige Bewegung des Sirups (zusätzlich zu dem vorstehend erwähnten Mischen), insbesondere durch durch Schlagstoß erzeugte mechanische Schocks in der Übersättigungszone,

d) Bewirken des Fortgangs der Kristallisation durch Anhalten der Verdampfung und der heftigen Bewegung (Schlagen) und Beibehalten einer regelmäßigen Bewegung (Mischen) zwischen der Zeit, die zum Erhalt von Kristallen mit der gewünschten Größe notwendig ist und vorzugsweise zwischen ungefähr 5 Minuten und ungefähr 20 Minuten,

e) Aufnehmen der Verdampfung (immer mit Mischen des Milieus mit einer Geschwindigkeit von ungefähr 100 bis 350 U/min) bis zum. Erhalt von Kristallen, die weniger als 1%, insbesondere weniger als 0,5% Feuchtigkeit enthalten, wobei die Temperatur bei einem Wert von ungefähr 70°C bis ungefähr 100°C gehalten wird, während der ganzen Dauer des Verfahrens und der Druck vorzugsweise bei ungefähr 200 mbar während den Schritten c) bis e) gehalten wird.

7. Verfahren zur Herstellung einer Zusammensetzung gemäß einem der Ansprüche 1 bis 3 und 5, das durch die folgenden Schritte **gekennzeichnet** ist:

a) Herstellen eines konzentrierten Zuckersirups von ungefähr 60% bis ungefähr 97%, insbesondere 75 Gew.-% der Trockenmasse,

b) Provozieren der Verdampfung des Sirups durch Vermindern des Drucks, so dass das Sieden des Sirups bei der ausgewählten Temperatur erreicht wird,

c) Mischen des Sirups, insbesondere durch mechanische Bewegung mit einer peripheren Geschwindigkeit von ungefähr 100 bis ungefähr 350 U/min, insbesondere 200 bis 350 U/min, wobei der Übersättigungskoeffizient des Sirups zwischen 1 und 1,3 umfasst, insbesondere zwischen 1,1 und 1,3, und Provozieren der Kristallisation durch heftige Bewegung des Sirups (zusätzlich zu dem vorstehend erwähnten Mischen), insbesondere durch durch Schlagstoß erzeugte mechanische Schocks in der Zone der Übersättigung,

d) Weiterführen der Verdampfung unter den gleichen Temperatur- und Druckbedingungen wie diejenigen, die in den vorhergehenden Schritten verwendet wurden, bis zum Erhalt eines Milieus, bei dem die Kristalle die Hauptphase zusammensetzen (mehr als ungefähr 50% und insbesondere mehr als ungefähr 70% in Bezug auf das Milieu), wobei die Bewegungsgeschwindigkeit um ungefähr 50 bis ungefähr 200 U/min vermindert wird, die Temperatur in Bezug auf die vorhergehenden Schritte konstant gehalten wird, das Schlagen bis zum Erhalt eines trockenen Produktes beibehalten wird, das aus Kristallen mit der gewünschten Größe zusammengesetzt ist, die weniger als 1%, insbesondere weniger als 0,5% Feuchtigkeit enthalten,

wobei die Temperatur auf einen konstanten Wert im Bereich von ungefähr 40°C bis ungefähr 100°C, insbesondere ungefähr 70°C bis ungefähr 100°C während der Dauer der Schritte eingestellt und gehalten wird.

Figure 1A

Figure 1B

Figure 1C

Figure 1D

# Vitesse de dissolution des sucres dans l'eau pure à 18°C

temps à la dissolution totale (sec.)

## FIG.2

EP 0 870 064 B1

# INDICE DE COULABILITE DES SUCRES

## FIG. 3

EP 0 870 064 B1

FIG.4